# EUROPEAN PATENT SPECIFICATION

(11) **EP 0 895 987 B1**
(45) Date of publication and mention of the grant of the patent: **31.03.2004**
(21) Application number: 98111951.4
(22) Date of filing: 29.06.1998
(51) Int. Cl.: C07C 253/30, C07C 255/23

(54) **Highly pure alkyl 2-cyanoacrylates**
Hochreine Alkyl-2-cyanoacrylate
2-Alkyl-cyanoacrylates de haute pureté

(30) Priority: 07.08.1997 EP 97113612
(43) Date of publication of application: 10.02.1999
(73) Proprietor: Cancel, Richard, 83130 La Garde (FR); Wallace, Richard, 83590 Gonfaron (FR); Sassi, Gérard, 83200 Toulon (FR)
(72) Inventor: Sailhan, Valérie, 34090 Montpellier (FR); Schue, François, Lab. de Chimie Macromoléculaire, 34090 Montpellier (FR); Eloy, Rosy, 38670 Chasse sur Rhône (FR); Giral, Louis, 34090 Montpellier (FR)
(74) Representative: Breese, Pierre

(56) References cited:
- DE-A- 2 104 518
- US-A- 5 455 369
- F. LEONARD ET AL.: "Synthesis and Degradation of Poly(alkyl-alpha-Cyanoacrylates)" JOURNAL OF APPLIED POLYMER SCIENCE., vol. 10, 1966, pages 259-272, XP002085470 NEW YORK US

## Description

The invention relates to a process for the preparation of a highly pure alkyl 2-cyanoacrylate,
wherein alkyl has 1-10 C atoms,
characterized in that
a) formaldehyde is reacted with an alkyl cyanoacetate ester by means of a catalyst in a solvent mixture selected from the group comprising ethylacetate, diglyme, dichloroethane and benzene,
   yielding an oligo-(alkyl 2-cyanoacrylate);
b) the product of (a) is separated in the form of a solid;
c) the product of (b) is depolymerized under sulfur dioxide atmosphere free from other polymerization inhibitors to yield an alkyl 2-cyanoacrylate with a purity of 98 - 100 %.

In 1951, cyanoacrylates were showed to possess good adhesive properties. The methyl cyanoacrylate was the first cyanoacrylate commercialized as adhesive in 1958, by the name of Eastman 910. Other cycanoacrylates broke through, such as Super glue, Krazy glue or Histoacryl Blue (butyl 2-cyanoacrylate).

Processes for the preparation of alkyl cyanoacrylates are known. A process by depolymerization of poly-α-alkyl cyanoacrylates and subsequent destillation of the monomers by means of special destillation columns is described in DE 36 38 171.
A depolymerization of poly-(alkyl cyanoacrylates) in presence of P₂O₅, hydroquinone and other additives like trikresylphosphate is described in DE 34 09 716.
Synthesis and degradation of poly-(alkyl α-Cyanoacrylates) is known by F. Leonard et al., J. Appl. Polymer Science, **10** (1966), 259-272. Leonard uses methanol as solvent for reacting formaldehyde with alkyl cyanoacetate esters.

The capability of alkyl cyanoacrylates to adhere firmly to most surfaces has evoked considerable medical interest.
In dental surgery cyanoacrylates can be used as glue for alloys or as adhesives as dental cement. Furthermore, they can be used in case of bone fractions.

Eastman 910 and Histoacryl blue, respectively based on methyl- and n-butyl cyanoacrylate, were used in surgery of the nose, ears, eyes, and skin. The compounds rapidly polymerize when applied on the surface of biological tissue.
However, the alkyl cyanoacrylates used for these purposes show some toxicity. This is the reason why nowadays these surgical adhesives are used only to strengthen sutures and in sprays as hemostatic agents.

Histoacryl Blue is a commercialized adhesive. It is not further used as a replacement for sutures in surgery anymore, as studies in vitro have pointed out a certain toxicity after the application of this compound. Consequently, surgeons are presently reluctant to use it (M. Forseth and K. O'Grady, The current status of cyanoacrylate and fibrin tissue adhesives, J. of Long-term Effects of Medical Implants, **1992,** 2(4), 221-233; F. Leonard, The n-alkyl alphacyanoacrylate tissue adhesives, Annales New York Academy of Sciences, **1985**, 203-213; Toxicity of alkyl 2-cyanoacrylates: I.Periphal nerve, Arch. Surg., **1966**, 93, 441-446).

This toxicity seems to be caused by the presence of impurities during the synthesis and preparation of the alkyl cyanoacrylates.
Furthermore, impurities initiiate polymerization of the obtained monomers and affect their stability.
The monomers obtained by processes of the prior art were found to have purities < 98 %.

The object of the invention was to discover an improved process for the preparation of alkyl cyanoacrylates which are highly pure.

Surprisingly, it has been found that the alkyl cyanoacrylates obtainable by the process acording to the invention show a purity higher than 98 %, generally the purities are between 99.00 and 100 %, in most cases between 99.98 and 100 %.

The scheme of synthesis is as follows:
1. oligomerization 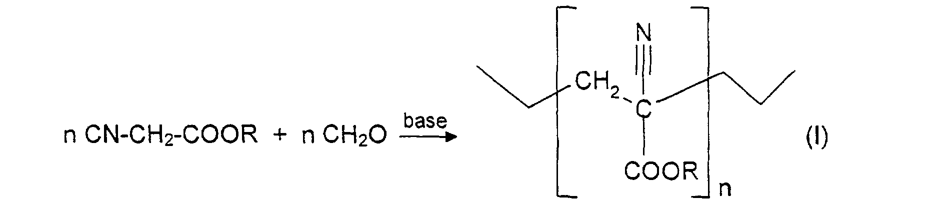
2. depolymerization

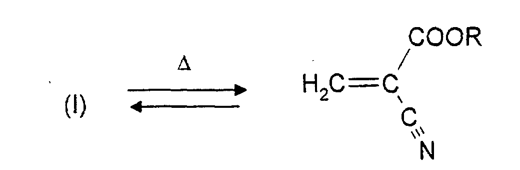

Alkyl cyanoacetates and (para-)formaldehyde are well known compounds. The preparation of alkyl cyanoacetates is carried out analogously to e.g. K. Tarui and S. Morimoto, Japan. 3418 (1956); Chem. Abstr. **51**, 13910i, by esterification of cyanoacetic acid with the corresponding alcohol.

Suitable solvents used in the first step are, for example, ethylacetate, diglyme, dichloroethane and benzene or mixtures of the solvents. Preferably, ethylacetate, dichloroethane or a mixture of ethylacetate/diglyme or dichloroethane/diglyme is used in the polymerization step.

Suitable catalysts are preferably bases such as piperidine.

The reaction expediently takes place at temperatures between 30 and 120°; it is preferably carried out between 40 and 110°, but particularly preferably between 70 and 110°.

With the oligomers obtained in the form of a solid in the first step, the depolymerization step can be carried without the need of the addition of the anion scavenger P₂O₅ or the addition other radical polymerization inhibitors.
The use of P₂O₅ and hydroquinone is described by Leonard et al. and in DE 34 09 716.

In contrast to what is known from prior art (DE 34 09 716), there is no necessity to add a free radical stabiliser such as hydroquinone to inhibit free radical polymerization during the depolymerization step.

The depolymerization expediently is carried out under (silicon-)bath temperatures between 100 und 250° C, preferably between 150 and 230°C, but most preferably between 160 and 210°.
The depolymerization is carried out at a pressure in the range of 10 Pa to 13 kPa, preferably between 20 Pa and 700 Pa, most preferably between 30 and 400 Pa, but is particularly preferably between 30 and 300 Pa (0.3 - 3 mbar).

The invention also relates to a process, wherein the polymerization reaction of formaldehyde with alkyl cyanoacetate esters expediently is carried out at temperatures between 10 and 150°, preferably between 20 and 130°, most preferably between 30 and 120° C.

Furthermore, the invention relates to a process, wherein the depolymerization is carried out under temperatures between 100 und 250° C. Additionally, the invention relates to a process, wherein the depolymerization is carried out at a pressure in the range of 10 Pa to 13 kPa.

Alkyl has 1-10 C atoms and preferably is methyl, ethyl, propyl, isopropyl, butyl, tert.-butyl, isobutyl, pentyl (amyl), isopentyl, hexyl, cyclohexyl, heptyl, octyl, nonyl or decyl.

The alkyl 2-cyanoacrylates can be used as adhesives.
Preferably, they can be used as surgical glue.
Furthermore, they can be used as coatings in pharmaceutical formulations, e.g. for incapsulation of medicaments, as described e.g. by C. Dubernet and J.P. Benoit, L'actualité chimique, 19-28 Décembre (1986).

Moreover, they can be used as resins in microlithography in manufacturing integral circuits.

Hereinbefore and hereinafter, all temperatures are indicated in °C. Determination of purity is determined by gas chromatography (GC) as follows:

| | condition I | condition II* |
|---|---|---|
| used column | megabore: -diameter =0.536 mm; -length = 15m; | megabore: -diameter =0.536 mm; -length = 15m; |
| | - stationnary phase : DB 225 - | stationnary phase : DB 225 |
| solvant | nitromethane or chloroforme (monomer 10 % w/w) | |
| temperature (column) | 150-170 | 170 (isotherm) |
| temperature (injection) | 220 | 220 |
| temperature (detector) | 250 | 250 |

| | | |
|---|---|---|
| * the alkyl 2-cyanoacrylate is purely injected | | |

### Example 1 (Comparative example)

n-hexyl 2-cyanoacrylate and n-butyl 2-cyanoacrylate have been prepared according to Leonard et al..
GC analysis under condition I reveals a peak of the solvent as well of the monomer.
The analyses confirm the purity values given by Leonard et al. being between 98.5 and 100 %.

Hoever, analysing the same compounds under GC condition II, the chromatogram shows additional peaks which were covered by the solvent peak under condition I.
Regarding the additional peaks n-hexyl 2-cyanoacrylate exhibits a purity of 96.03 % and n-butyl 2-cyanoacrylate shows a value of 97.67 %.

### Example 2

3.48 g (0.11g mol) of paraformaldehyde and 0.33 ml of piperidine are added to a solution of 12.5 ml of benzene and 12.5 ml of diglyme at about 50°.
19.32 g (0.1157 mol) of n-butyl cyanoacetate is added dropwise at about 90° (reflux condition). After the azeotropic separation of the water the solvents are removed by distillation under reduced pressure (1.33 hPa) without adding phosphorous pentaoxide.
The viscous residue is dissolved in warm methanol. After 12 hours at -18° the solid oligo-(n-butyl 2-cyanoacrylate) is separated by filtration and dried.

The depolymerization is carried out in a completely dried apparatus under sulfur dioxide atmosphere (purity of SO₂ = 99 %). The solid oligomer is heated at 170° under reduced pressure [50 Pa (0.5 mbar)]. The monomer n-butyl 2-cyanoacrylate is collected in a cooled receiver; b.p. 80°_{0.5 mbar}; purity (GC, condition II) 99.5 %.

The following alkyl 2-cyanoacrylates are obtained analogously:
n-hexyl 2-cyanoacrylate, b.p. 90°_{0.5 mbar}; purity (GC, condition II) 99.99%;
isobutyl 2-cyanoacrylate, b.p. 65°_{0.5 mbar}; purity (GC, condition II) 99.99%;
isoamyl 2-cyanoacrylate, b.p. 73°_{0.5 mbar}; purity (GC, condition II) 99.6%;
n-amyl 2-cyanoacrylate, b.p. 70°_{0.5 mbar}; purity (GC, condition II) 99.99%;
cyclohexyl 2-cyanoacrylate, b.p. 100°_{0.5 mbar}; purity (GC, condition II) 99.99%.

### Example 3

n-Butyl 2-cyanoacrylate was obtained analogously to Example 2, except that benzene was replaced by ethyl acetate; purity (GC, condition II) 99.99 %.

## Claims

1. A process for the preparation of a highly pure alkyl 2-cyanoacrylate, wherein alkyl has 1-10 C atoms, **characterized in that** :
a) formaldhehyde is reacted with an alkyl cyanoacetate ester by means of a catalyst in a suitable solvent selected from the group comprising ethylacetate, diglyme, dichloroethane, and benzene or a mixture thereof yielding an oligo-(alkyl 2-cyanoacrylate) ;
b) the product of (a) is separated in the form of a solid ;
c) the product of (b) is depolymerized under sulfur dioxide atmosphere free from other polymerization inhibitors to yield an alkyl 2-cyanoacrylate with a purity of 98 - 100 %.

2. A process according to claim 1, wherein the suitable solvent is a solvent mixture of ethylacetate and diglyme.

3. A process according to claim 1, wherein the suitable solvent is a solvent mixture of dichloroethane and diglyme.

4. A process according to any of claims 1 to 3, wherein the polymerization reaction of formaldehyde with alkyl cyanoacetate esters is carried out at temperatures between 30 and 120 °C.

5. A process according to any of claims 1 to 4, wherein the depolymerization is carried out under temperatures between 100 and 250 °C.

6. A process according to any of claims 1 to 5, wherein the depolymerization is carried out at a pressure in the range of 10 Pa to 13 kPa.

## Patentansprüche

1. Verfahren zur Herstellung eines hochreinen Alkyl-2-cyanacrylats, wobei das Alkyl 1-10 C-Atome aufweist,
**dadurch gekennzeichnet, dass**
a) Formaldehyd mit einem Alkylcyanacetatester mit Hilfe eines Katalysators in einer Mischung aus Lösungsmitteln ausgewählt aus der Gruppe bestehend aus Ethylacetat, Diglyme, Dichlorethan und Benzol oder einer Mischung davon umgesetzt wird, was ein Oligo-(alkyl-2-cyanacrylat) ergibt;
b) das Produkt aus (a) in Form eines Feststoffs abgetrennt wird;
c) das Produkt aus (b) unter Schwefeldioxidatmosphäre, die frei von anderen Polymerisationshemmern ist, entpolymerisiert, was ein Alkyl-2-cyanacrylat mit einer Reinheit von 98 - 100 % ergibt.

2. Verfahren nach Anspruch 1, wobei ein geeignetes Lösungsmittel eine Mischung aus den Lösungsmitteln Ethylacetat und Diglyme darstellt.

3. Verfahren nach Anspruch 1, wobei ein geeignetes Lösungsmittel eine Mischung aus den Lösungsmitteln Dichlorethan und Diglyme darstellt.

4. Verfahren nach einem der Ansprüche 1 bis 3, wobei die Polymerisationsreaktion von Formaldehyd und Alkylcyanacetatestern bei Temperaturen zwischen 30 und 120 °C durchgeführt wird.

5. Verfahren nach einem der Ansprüche 1 bis 4, wobei die Depolymerisation bei Temperaturen zwischen 100 und 250 °C durchgeführt wird.

6. Verfahren nach einem der Ansprüche 1 bis 5, wobei die Depolymerisation bei einem Druck im Bereich von 10 Pa bis 13 kP durchgeführt wird.

## Revendications

1. Procédé pour la préparation d'un 2-cyanoacrylate d'alkyle extrèmement pur, dans lequel l'alkyle a 1 à 10 atomes de carbone, **caractérisé en ce que** :
a) le formaldéhyde est mis en réaction avec un ester de cyanoacétate d'alkyle au moyen d'un catalyseur dans un solvant approprié choisi dans le groupe comprenant l'acétate d'éthyle, le diglyme, le dichloroéthane et le benzène ou un mélange de ceux-ci donnant un oligo-(2-cyanoacrylate d'alkyle) ;
b) le produit de (a) est séparé sous la forme d'un solide ;
c) le produit de (b) est dépolymérisé sous atmosphère de dioxyde de soufre, libre de tout autre inhibiteur de polymérisation pour donner un 2-cyanoacrylate d'alkyle avec une pureté comprise entre 98 % et 100 %.

2. Procédé selon la revendication 1, dans lequel le solvant approprié est un mélange de solvants d'acétate d'éthyle et de diglyme.

3. Procédé selon la revendication 1, dans lequel le solvant approprié est un mélange des solvants dichloroéthane et diglyme.

4. Procédé selon l'une quelconque des revendications 1 à 3, dans lequel la réaction de polymérisation du formaldéhyde avec les esters de cyanoacétate d'alkyle est réalisée à des températures comprises entre 30 °C et 120 °C.

5. Procédé selon l'une quelconque des revendications 1 à 4, dans lequel la dépolymérisation est réalisée sous des températures comprises entre 100 °C et 250 °C.

6. Procédé selon l'une quelconque des revendications 1 à 5, dans lequel la dépolymérisation est réalisée à une pression comprise dans la plage allant de 10 Pa à 13 KPa.
